Europäisches Patentamt

European Patent Office

Office européen des brevets

(19)

(11) EP 0 548 356 B1

## (12) FASCICULE DE BREVET EUROPEEN

(45) Date de publication et mention
de la délivrance du brevet:
**29.01.1997 Bulletin 1997/05**

(21) Numéro de dépôt: **92917134.6**

(22) Date de dépôt: **21.07.1992**

(51) Int. Cl.$^6$: **A61K 9/20**

(86) Numéro de dépôt international:
**PCT/FR92/00715**

(87) Numéro de publication internationale:
**WO 93/01805 (04.02.1993 Gazette 1993/04)**

(54) **COMPRIME MULTIPARTICULAIRE A DELITEMENT RAPIDE**

MULTIPARTIKEL-TABLETTE MIT SCHNELLAUFLÖSBARKEIT

RAPIDLY DISINTEGRATABLE MULTIPARTICULATE TABLET

(84) Etats contractants désignés:
**AT BE CH DE DK ES FR GB GR IT LI LU MC NL
SE**

(30) Priorité: **22.07.1991 FR 9109245**

(43) Date de publication de la demande:
**30.06.1993 Bulletin 1993/26**

(73) Titulaire: **LABORATOIRES PROGRAPHARM
28170 Châteauneuf-en-Thymerais (FR)**

(72) Inventeurs:
• **COUSIN, Gérard
F-28320 Gallardon (FR)**

• **BRUNA, Etienne
F-28000 Chartres (FR)**
• **GENDROT, Edouard
F-28500 Vernouillet (FR)**

(74) Mandataire: **Koch, Gustave et al
Cabinet PLASSERAUD
84, rue d'Amsterdam
75440 Paris Cédex 09 (FR)**

(56) Documents cités:
**EP-A- 0 255 002     EP-A- 0 281 200
EP-A- 0 408 273**

**Description**

L'invention a pour objet un comprimé multiparticulaire à délitement rapide qui constitue une forme galénique pour administration orale et dont la vitesse de délitement est telle que, lorsqu'il est placé dans la cavité buccale et notamment sur la langue, il se désagrège en moins de 60 secondes pour fournir avec la salive présente une suspension aisée à avaler.

La vitesse de délitement est obtenue grâce à un mélange d'excipients qui comporte généralement un agent désintégrant pouvant être constitué par une carboxyméthylcellulose et un agent gonflant pouvant être constitué par de l'amidon modifié.

La substance active est mélangée aux excipients dont il vient d'être question, l'ensemble étant mis sous forme de comprimé après addition d'un agent lubrifiant tel que, par exemple, le stéarate de magnésium.

La Société Demanderesse a le mérite d'avoir trouvé qu'il était possible, de façon inattendue et surprenante, de faire comporter à un comprimé multiparticulaire à délitement rapide tel que défini plus haut, la substance active sous la forme de microcristaux enrobés ou de microgranules enrobés ou non; ainsi, le praticien dispose d'un comprimé multiparticulaire à délitement rapide propre à faciliter l'absorption par le patient de substances actives les plus diverses et notamment celles dont le goût est particulièrement désagréable et de permettre l'absorption desdites substances actives sous des caractéristiques aussi diverses que la gastrorésistance et la libération contrôlée, les microcristaux enrobés et les microgranules enrobés ou non conservant, après mise sous forme de comprimé multiparticulaire, leurs propriétés initiales dont notamment le masquage du goût, la gastrorésistance et la libération contrôlée du principe actif.

En conséquence le comprimé multiparticulaire conforme à l'invention pour administration orale avec ou sans utilisation d'eau, dont la vitesse de délitement entraîne la désagrégation en moins de 60 secondes et qui comprend une substance active et un mélange d'excipients comprenant un ou plusieurs agents désintégrants et/ou un ou plusieurs agents gonflants ou solubles, est caractérisé par le fait que la substance active est multiparticulaire et se présente sous la forme de microcristaux enrobés ou de microgranules enrobés.

Selon un mode de réalisation avantageux du susdit comprimé, le mélange d'excipients comprend un ou plusieurs agents désintégrants du type carboxyméthylcellulose ou PVP réticulé insoluble, un ou plusieurs agents gonflants pouvant être constitués par une carboxyméthylcellulose, un amidon, un amidon modifié, par exemple un amidon carboxyméthylé, ou une cellulose microcristalline, et éventuellement un sucre de compression directe constitué par exemple de 92% de dextrose.

Selon un mode de réalisation avantageux, les comprimés conformes à l'invention, dans lesquels la substance active est présente sous forme de microcristaux enrobés, comportent, à titre de substance active, au moins l'une de celles du groupe comprenant les sédatifs gastrointestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

Selon un autre mode de réalisation avantageux, les comprimés conformes à l'invention, dans lesquels la substance active est présente sous la forme de microgranules enrobés ou non à action modifiée ou non, comportent, à titre de substance active, au moins l'une de celles du groupe comprenant les sédatifs gastro-intestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la cohabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

L'usage du comprimé conforme à l'invention est particulièrement pratique du fait qu'il est d'une très grande facilité d'utilisation pour tous les utilisateurs. Il peut être pris dans toutes conditions (travail, voyage et autres), sans verre ni eau. C'est une forme pharmaceutique "ambulatoire" qui peut remplacer avantageusement de nombreuses formes pharmaceutiques telles que les sachets, comprimés effervescents, ampoules buvables, gélules, comprimés traditionnels et autres.

Sa grande facilité d'administration est particulièrement intéressante lorsqu'il s'agit de faire absorber une substance thérapeutique par de jeunes enfants ou des personnes âgées, populations présentant souvent des difficultés de déglutition, c'est-à-dire conservant le médicament dans la bouche sans arriver à l'avaler. Contrairement au comprimé traditionnel ou à la gélule, le comprimé conforme à l'invention présente chez ces sujets un avantage de sécurité car, dès son introduction dans la bouche, il offre une protection thérapeutique.

D'autre part, il est important de souligner que, même avalé directement avec l'aide d'un peu d'eau par exemple, ce comprimé conserve sa rapidité de délitement dans l'estomac. Ce type d'administration ne poserait donc, lui non plus, aucun problème de sécurité.

De plus, le comprimé faisant l'objet de l'invention présente un grand avantage par rapport aux comprimés ou gélules simples. En effet, jusqu'à ce jour, les personnes devant avaler un comprimé ou une gélule dans des conditions telles que celles évoquées ci-dessus (travail, voyage, sans eau ni verre), l'avalaient sans eau, ce qui est dangereux car alors le comprimé ou gélule peut se bloquer dans l'oesophage et causer ainsi un retard important dans l'absorption du principe actif ou même une ulcération au niveau de l'oesophage. De même, le fait que, d'une part, le principe actif soit enrobé et, d'autre part, qu'il se présente sous forme multiparticulaire, empêche les principes actifs agressifs de provoquer des ulcérations de la muqueuse oesophagienne ou gastrique, phénomène parfois causé par certaines formes pharmaceutiques monolithiques, surtout lorsque le patient parvient à les avaler avec peu ou pas d'eau.

Un autre avantage réside dans le fait que le comprimé conforme à l'invention ne présente pas les inconvénients bien connus des comprimés effervescents qui sont par exemple le goût qui est très désagréable à l'enfant, la teneur élevée en sodium qui est gênante pour les patients soumis à un régime désodé et la nécessité de disposer d'eau et d'un verre pour son administration.

De plus, il permet la formulation de certains principes actifs qui ne se prêtent pas à une dissolution extracorporelle préalable et qui ne peuvent donc être envisagés que sous forme sèche, ce qui exclut leur emploi dans les comprimés effervescents; par conséquent, le comprimé de la présente invention présente tous les avantages des formes sèches, à savoir la stabilité et la facilité de conditionnement et de conservation.

D'autre part, cette nouvelle forme pharmaceutique peut éventuellement contenir deux ou plusieurs principes actifs habituellement incompatibles sans altérer leur stabilité.

Un autre avantage du comprimé conforme à l'invention est de permettre l'ingestion par le patient de doses de principe actif plus importantes que par le passé. En effet, le comprimé ne devant pas être avalé dans sa forme initiale mais après délitement dans la cavité buccale, sa taille peut être supérieure à celle d'une forme pharmaceutique classique devant être avalée sans gêner la prise du médicament.

Enfin, le comprimé conforme à l'invention présente tous les avantages des particules enrobées permettant d'obtenir notamment un masquage de goût, un caractère gastro-résistant, une libération prolongée ainsi que tous les avantages des formes multiparticulaires à action modifiée ou non, à savoir une grande surface d'échange, la dispersion, moins de variations inter- et intra-individuelles, un effet très atténué de la vidange gastrique, du temps de transit intestinal, du pH dans le tube digestif, de la viscosité et donc de la nourriture et de la position du corps, sans manifestations toxiques locales.

Pour préparer les comprimés multiparticulaires à délitement rapide conformes à l'invention, on procède comme suit ou de façon analogue.

Dans le cas où la substance active est présente sous forme de microcristaux enrobés, on peut procéder comme indiqué ci-après.

Les microcristaux sont enrobés par un procédé en lui-même connu tel que, par exemple, le procédé en lit d'air fluidisé, la coacervation, la microencapsulation.

Le mélange d'excipients est alors préparé par granulation préalable par voie sèche ou humide.

Puis, les microcristaux enrobés sont mélangés à sec avec le mélange d'excipients avant d'être comprimés.

Pour la préparation du comprimé conforme à l'invention dans lequel la substance active est présente sous forme de microgranules enrobés ou non, on peut procéder comme indiqué ci-après.

Le principe actif est mis sous forme de microgranules par un procédé en lui-même connu tel que, par exemple, l'extrusion-sphéronisation, la fabrication en turbine, le lit d'air fluidisé et autres.

Une fois obtenus, ces microgranules sont éventuellement enrobés en turbine ou lit d'air fluidisé.

Le mélange d'excipients est alors préparé par granulation préalable par voie sèche ou humide.

Puis, les microgranules enrobés ou non sont mélangés à sec avec le mélange d'excipients avant d'être comprimés.

L'invention pourra être mieux comprise à l'aide des exemples qui suivent et qui sont relatifs à des modes de réalisation avantageux de l'invention.

## EXEMPLE 1

### Comprimé multiparticulaire à délitement rapide à base de cristaux enrobés de paracétamol.

On se propose de préparer des comprimés conformes à l'invention constitués comme suit.

| Formule: | |
|---|---|
| paracétamol enrobé (dont 6% d'éthylcellulose) | 530 mg |
| sucre de compression directe | 160 mg |
| cellulose microcristalline | 90 mg |
| polyvinylpyrrolidone réticulée | 60 mg |
| carboxyméthylcellulose sodique | 50 mg |
| silice colloïdale | 6 mg |
| lubrifiant | 4 mg |
| édulcorant | 25 mg |
| arômes | 15 mg |
| trisilicate de magnésium | 50 mg |
| Total | 990 mg |

Pour préparer ce comprimé, on procède comme suit.

On introduit les cristaux de paracétamol dans un appareil à lit d'air fluidisé et on pulvérise une solution d'éthylcellulose dans un mélange éthanol/acétone.

On tamise tous les excipients et on homogénéise le paracétamol enrobé avec les excipients dans un mélangeur à sec.

On procède à la répartition et à la mise en forme sur comprimeuse équipée de poinçons de diamètre 15 mm et rayon de courbure 20 mm.

La pression exercée est de 16 KNewtons ±1. La dureté des comprimés obtenus est de 100 Newtons ±10. Le temps de désagrégation dans la bouche est de 35 à 45 secondes.

## EXEMPLE 2

### Comprimé multiparticulaire à délitement rapide à base de cristaux enrobés de cimétidine.

On se propose de préparer des comprimés conformes à l'invention constitués comme suit.

| Formule: | |
|---|---|
| cimétidine enrobée (dont 15,25% d'Eudragit E) | 944 mg |
| polyvinylpyrrolidone réticulée | 89 mg |
| stéarate de magnésium | 5 mg |
| édulcorant | 50 mg |
| arômes | 12 mg |
| Total | 1100 mg |

Pour préparer ce comprimé, on procède comme suit.

On introduit les cristaux de cimétidine dans un appareil à lit d'air fluidisé et on pulvérise une solution d'un copolymère de diméthyl-aminoéthyl-méthacrylate et d'esters neutres de l'acide méthacrylique connu sous la dénomination "Eudragit E" dans l'alcool.

4

On tamise tous les excipients et on homogénéise la cimétidine enrobée avec les excipients dans un mélangeur à sec.

On procède à la répartition et à la mise en forme sur comprimeuse équipée de poinçons de diamètre 16 mm et rayon de courbure 20 mm.

La pression exercée est de 20 KNewtons ±1. La dureté des comprimés obtenus est de 95 Newtons ±10. Le temps de désagrégation dans la bouche est de 15 à 20 secondes.

### EXEMPLE 3

**Comprimé multiparticulaire à délitement rapide à base de cristaux enrobés de paracétamol.**

On se propose de préparer des comprimés conformes à l'invention constitués comme suit.

| Formule: | |
|---|---|
| complexe paracétamol-codéine (30 mg de codéine et 18,4% d'Eudragit*) | 627,5 mg |
| polyvinylpyrrolidone réticulée | 90 mg |
| carboxyméthylcellulose sodique | 70 mg |
| amidon commercialisé sous la dénomination "STARCH 1500" | 100 mg |
| édulcorant | 40 mg |
| arômes | 22,5 mg |
| Total | 950 mg |

    * L'Eudragit est un copolymère de l'acide mèthacrylique. Pour prèparer ce comprimè, on
    procède comme suit.

On introduit les cristaux de paracétamol dans un appareil à lit d'air fluidisé et on pulvérise de la codéine dans une solution d'Eudragit E et Eudragit NE 30D (polymère neutre d'esters de l'acide polyméthacrylique).

On tamise tous les excipients et on homogénéise le paracétamol enrobé avec les excipients dans un mélangeur à sec.

On procède à la répartition et à la mise en forme sur comprimeuse équipée de poinçons de diamètre 16 mm et rayon de courbure 20 mm.

La pression exercée est de 21 KNewtons ±1. La dureté des comprimés obtenus est de 35 Newtons ±5. Le temps de désagrégation dans la bouche est de 50 à 60 secondes.

### EXEMPLE 4

**Comprimé multiparticulaire à délitement rapide à base de cristaux enrobés d'ibuprofène.**

On se propose de préparer des comprimés conformes à l'invention constitués comme suit.

| Formule: | |
|---|---|
| ibuprofène (dont 10% d'éthylcellulose) | 440 mg |
| polyvinylpyrrolidone réticulée | 120 mg |
| amidon commercialisé sous la dénomination "STARCH 1500" | 235 mg |
| édulcorant | 48 mg |
| arômes | 52 mg |
| stéarate de magnésium | 5 mg |
| Total | 900 mg |

Pour préparer ce comprimé, on procède comme suit.

On introduit les cristaux d'ibuprofène dans un appareil à lit d'air fluidisé et on pulvérise une solution d'éthylcellulose dans l'éthanol.

On tamise tous les excipients et on homogénéise l'ibuprofène enrobé avec les excipients dans un mélangeur à sec.

On procède à la répartition et à la mise en forme sur comprimeuse équipée de poinçons de diamètre 16 mm et rayon de courbure 20 mm.

La pression exercée est de 15 KNewtons ±1. La dureté des comprimés obtenus est de 50 Newtons ±5. Le temps de désagrégation dans la bouche est de 15 à 20 secondes.

## EXEMPLE 5

**Comprimé multiparticulaire à délitement rapide à base de microgranules.**

| Formule: | |
| --- | --- |
| microgranules à libération prolongée de doxycycline monohydrate (dont 100 mg de principe actif) | 225 mg |
| cellulose microcristalline | 142 mg |
| amidon commercialisé sous la dénomination "SEPPISTAB ST 500" | 98 mg |
| aspartam | 20 mg |
| arômes | 15 mg |
| Total | 500 mg |

On fabrique les microgranules par montage de la doxycycline en turbine à partir d'un grain neutre selon la technologie classique, les microgranules étant ensuite enrobés à l'aide d'Eudragit E en turbine.

On fabrique le comprimé par tamisage de tous les excipients, suivi d'une homogénéisation des microgranules de doxycycline avec les excipients dans un mélangeur à sec, puis d'une répartition et mise en forme sur comprimeuse rotative équipée de poinçons de diamètre 12 mm et rayon de courbure 11 mm.

La pression exercée est de 20 KNewtons ±1. La dureté des comprimés obtenus est de 100 Newtons ±10. Le temps de désagrégation dans la bouche est de 10 à 20 secondes.

En suite de quoi on dispose d'un comprimé multiparticulaire à délitement rapide dont la constitution et le procédé de fabrication résultent suffisamment de ce qui précède pour qu'il soit inutile d'insister à ce sujet et à propos duquel il est rappelé que

- c'est un comprimé qui allie l'apport d'une technologie de haut niveau (contrôle de la libération, de la gastrorésistance, masquage du goût du principe actif) à une grande sécurité d'utilisation due à sa forme multiparticulaire par l'intervention de l'enrobage dans son procédé de fabrication et au fait que son délitement soit assuré dans la cavité buccale,
- il constitue et permet une forme ambulatoire pouvant être adaptée à un grand nombre de principes actifs et à de forts dosages, ce qui auparavant n'existait pas,
- il offre une grande souplesse d'utilisation, la même forme pharmaceutique pouvant être délitée dans la bouche, dans un verre d'eau ou dans de la nourriture liquide ou semi-liquide, comme par exemple dans le yaourt pour les enfants ou nourrissons, ou dans la nourriture animale dans le cas d'un usage vétérinaire,
- il constitue une seule et même forme pharmaceutique qui peut être prescrite à des personnes nécessitant des doses tout à fait variées; en effet, il peut être utilisé pour un principe actif donné dans son dosage maximal et être fabriqué de manière à être sécable en un ou plusieurs endroits de façon à pouvoir être administré dans sa totalité ou selon l'âge et les symptômes du patient, sous forme d'une partie divisible fonction de la forme du poinçon, étant souligné que le fait de pouvoir obtenir un comprimé multiparticulaire sécable n'était pas évident,
- il constitue, par conséquent, une forme pharmaceutique pouvant convenir à tous car elle offre une grande variété de moyens d'administration et de dosages, ce qui présente un avantage économique certain.

Le fait qu'un seul produit puisse permettre, d'une part, des modes d'administration divers habituellement permis par plusieurs formes pharmaceutiques et, d'autre part, puisse générer plusieurs posologies habituellement obtenues en créant divers dosages (comprimés ou gélules de différentes concentrations par exemple) est un avantage économique primordial.

En effet, au niveau industriel, cela se traduit par une seule ligne de fabrication au lieu de plusieurs lignes de fabrication correspondant chacune à chaque dosage et à chaque forme pharmaceutiques retenus.

## Revendications

1. Comprimé multiparticulaire pour administration orale avec ou sans utilisation d'eau, dont la vitesse de délitement entraîne la désagrégation en moins de 60 secondes et qui comprend une substance active et un mélange d'excipients comprenant un ou plusieurs agents désintégrants et/ou un ou plusieurs agents gonflants ou solubles, caractérisé par le fait que la substance active est multiparticulaire et se présente sous la forme de microcristaux enrobés ou de microgranules enrobés.

2. Comprimé selon la revendication 1, caractérisé par le fait que le mélange d'excipients comprend un ou plusieurs agents de désintégration du type carboxyméthylcellulose ou PVP réticulé insoluble, un ou plusieurs agents gonflants du type amidon, amidon modifié ou cellulose microcristalline et éventuellement un sucre de compression directe.

3. Comprimé selon l'une des revendications 1 et 2, caractérisé par le fait qu'il peut être délité dans la bouche, dans un verre d'eau ou dans de la nourriture liquide ou semi-liquide, comme par exemple du yaourt lorsqu'il s'agit d'un usage pédiatrique ou de la nourriture pour animaux lorsqu'il s'agit d'un usage vétérinaire.

4. Comprimé selon l'une des revendications 1 à 3, caractérisé par le fait qu'il comporte, sous forme de microcristaux enrobés, au moins l'une des substances du groupe comprenant les sédatifs gastro-intestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

5. Comprimé selon l'une des revendications 1 à 3, caractérisé par le fait qu'il comporte, sous forme de microgranules enrobés, au moins l'une des substances du groupe comprenant les sédatifs gastro-intestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiepileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

## Claims

1. Multiparticulate tablet for oral administration with or without the use of water, the disintegration speed of which causes the disintegration in less than sixty seconds, said tablet comprising an active substance and a mixture of excipients comprising one or several disintegrant agents and/or one or several swelling or soluble agents, characterized by the fact that the active substance is multi-particulate and in the form of coated microcrystals or coated microgranules.

2. Tablet according to claim 1, characterized by the fact that the mixture of excipients comprises one or several disintegration agents of the carboxymethylcellulose or insoluble reticulated PVP type, one or several swelling agents of the starch, modified starch or microcrystalline cellulose type and possibly a direct compression sugar.

3. Tablet according to one of claims 1 and 2, characterized by the fact that it can be disintegrated in the mouth, in a glass of water or in liquid or semi-liquid food, as for example in yoghourt in connection with its use in the paediatric field, or in food for animals in connection with its use in the veterinary field.

4. Tablet according to one of claims 1 to 3, characterized by the fact that it comprises, in the form of coated microcrystals, at least one of the substances of the group comprising the gastrointestinal sedatives, the antacids, the analgesics, the anti-inflammatory agents, the coronary vasodilators, the peripheral and brain-vasodilators, the anti-infectious agents, the antibiotics, the antiviral agents, the antiparasitic agents, the anticancerous drugs, the antianxiety agents, the neuroleptic drugs, the agents stimulating the central nervous system, the antidepressant drugs, the antihistaminic agents, the antidiarrheal agents, the laxatives, the nutritional supplements, the immuno-depressant drugs, the cholesterol lowering agents, the hormones, the enzymes, the antispasmodic agents, the antiangorous agents, the drugs acting on the rhythm of the heart, the drugs used in the treatment of arterial hypertension, the anti-migraine agents, the drugs acting on blood coagulability, the antiepileptic agents, the myorelaxing agents, the drugs used in the treatment of diabetes, the drugs used in the treatment of thyroidal dysfunctions, the diuretical agents, the anorexigenic drugs, the antiasthmatic agents, the expectorants, the antitussive agents, the mucoregulators, the decongestants, the hypnotics, the antinauseous agents, the hematopoietical agents, the uricosuric agents, the plant extracts, the contrast mediums.

5. Tablet according to one of claims 1 to 3, characterized by the fact that it comprises, in the form of coated microgranules, at least one of the substances of the group comprising the gastrointestinal sedatives, the antacids, the analgesics, the anti-inflammatory agents, the coronary vasodilators, the peripheral and brain-vasodilators, the anti-infectious agents, the antibiotics, the antiviral agents, the antiparasitic agents, the anticancerous drugs, the antianxiety agents, the neuroleptic drugs, the agents stimulating the central nervous system, the antidepressant drugs, the antihistaminic agents, the antidiarrheal agents, the laxatives, the nutritional supplements, the immuno-depressant drugs, the cholesterol lowering agents, the hormones, the enzymes, the antispasmodic agents, the antiangorous agents, the drugs acting on the rhythm of the heart, the drugs used in the treatment of arterial hypertension, the anti-migraine agents, the drugs acting on blood coagulability, the antiepileptic agents, the myorelaxing agents, the drugs used in the treatment of diabetes, the drugs used in the treatment of thyroidal dysfunctions, the diuretical agents, the anorexigenic drugs, the antiasthmatic agents, the expectorants, the antitussive agents, the mucoregulators, the decongestants, the hypnotics, the antinauseous agents, the hematopoietical agents, the uricosuric agents, the plant extracts, the contrast mediums.

**Patentansprüche**

1. Multipartikel-Tablette zur oralen Anwendung mit oder ohne Verwendung von Wasser, wobei die Zerfallgeschwindigkeit einen Zerfall in weniger als 60 Sekunden bewirkt und die einen Wirkstoff und eine Mischung von Zusatzstoffen enthalt, umfassend eine oder mehrere den Zerfall bewirkende Substanzen und/oder eine oder mehrere Treibmittel oder Lösungsmittel,
dadurch gekennzeichnet,
daß der Wirkstoff multipartikulär ist und in Form von umhüllten Mlkrokristallen oder von umhüllten Mikrokörnern aufgebaut ist.

2. Tablette nach Anspruch 1,
dadurch gekennzeichnet,
daß die Mischung der Zusatzstoffe einen oder mehrere den Zerfall bewirkende Substanzen vom Typ der Carboxymethylcellulose oder von unlöslich vernetztem PVP, ein oder mehrere Treibmittel vom Stärkemehltyp, modifiziertes Stärkemehl oder mikrokristalline Cellulose und gegebenenfalls einen Zucker zum direkten Verpressen umfaßt.

3. Tablette nach einem der Ansprüche 1 und 2,
dadurch gekennzeichnet,
daß sie im Mund, in einem Wasserglas oder in flüssigen oder halbflüssigen Lebensmitteln, wie z.B. Joghurt, insoweit es sich um pediatrische Verwendung handelt oder Futtermittel für Tiere, insoweit es sich um eine veterinäre Anwendung handelt, aufgelöst werden kann.

4. Tablette gemäß einem der Ansprüche 1 bis 3,
dadurch gekennzeichnet,
daß sie mindestens eine der Substanzen der Gruppe umfassend gastrointestinal Sedativa, Antacida, Analgetika, Entzündungshemmer, koronare Vasodilatatoren, periphere und zerebrale Vasodilatatoren, Antiinfektiva, Antibiotika, Antivirusmittel, Antiparasitenmittel, Antikrebsmittel, Anxiolytika, Neuroleptika, Stimulantien des zentralen Nervensystems, Antidepressiva, Antihistaminika, Mittel gegen Diarrhoe, Laxantien, Nahrungsergänzungsmittel, Immundepressiva, Mittel gegen Hypocholesterinemie, Hormone, Enzyme, krampflösende Mittel, Mittel gegen Angina pectoris, Medikamente zur Beeinflussung des Herzrythmus, Medikamente, die zur Behandlung von arterieller Hypertonie verwendet werden, Antimigränemittel, Medikamente, die die Blutgerinnung beeinflussen, Antiepileptika, Muskelrelaxantien, Medikamente, die zur Behandlung von Diabetes verwendet werden, Medikamente, die zur Behandlung von Schilddrüsenfehlfunktionen verwendet werden, Diuretika, Appetitzügler, Asthmamittel, schleimlösende Mittel, Hustenmittel, schleimregulierende Mittel, Dekongestiva, Hypnotika, Mittel gegen Übelkeit, blutbildende Mittel, Harnsäure ausscheidende Mittel, Pflanzenextrakte, Kontrastmittel in Form von umhüllten Mikrokristallen beinhaltet.

5. Tablette nach einem der Ansprüche 1 bis 3,
dadurch gekennzeichnet,
daß sie mindestens eine der Substanzen der Gruppe umfassend gastrointestinal Sedativa, Antacida, Analgetika, Entzündungshemmer, koronare Vasodilatatoren, periphere und zerebrale Vasodilatatoren, Antiinfektiva, Antibiotika, Antivirusmittel, Antiparasitenmittel, Antikrebsmittel, Anxiolytika, Neuroleptika, Stimulantien des zentralen Nervensystems, Antidepressiva, Antihistaminika, Mittel gegen Diarrhoe, Laxantien, Nahrungsergänzungsmittel, Immundepressiva, Mittel gegen Hypocholesterinemie, Hormone, Enzyme, krampflösende Mittel, Mittel gegen Angina pectoris, Medikamente zur Beeinflussung des Herzrythmus, Medikamente, die zur Behandlung von arterieller Hypertonie verwendet werden, Antimigränemittel, Medikamente, die die Blutgerinnung beeinflussen, Antiepileptika, Muskelrelaxantien, Medikamente, die zur Behandlung von Diabetes verwendet werden, Medikamente, die zur Behandlung von Schilddrüsenfehlfunktionen verwendet werden, Diuretika, Appetitzügler, Asthmamittel, schleimlösende Mittel, Hustenmittel, schleimregulierende Mittel, Dekongestiva, Hypnotika, Mittel gegen Übelkeit, blutbildende Mittel, Harnsäure ausscheidende Mittel, Pflanzenextrakte, Kontrastmittel in Form von umhüllten Mikrokörnern beinhaltet.